# FASCICULE DE BREVET EUROPEEN

(11) **EP 2 488 149 B1**
(45) Date de publication et mention de la délivrance du brevet: **02.10.2013**
(21) Numéro de dépôt: 10785466.3
(22) Date de dépôt: 13.10.2010
(51) Int. Cl.: A61K 8/11, A61K 8/26, A61K 8/64, A61Q 19/08, A61K 8/97

(54) **COMPOSITION DERMATOLOGIQUE ET/OU COSMETIQUE UTILISEE POUR LA REGENERATION DE LA PEAU**
DERMATOLOGISCHE UND/ODER KOSMETISCHE ZUSAMMENSETZUNG ZUR HAUTREGENERATION
DERMATOLOGICAL AND/OR COSMETIC COMPOSITION USED TO REGENERATE SKIN

(30) Priorité: 14.10.2009 FR 0957201
(43) Date de publication de la demande: 22.08.2012
(73) Titulaire: Laboratoire Cosmetique De Lecousse, 75015 Paris (FR)
(72) Inventeur: DELAUNAY, Dominique, F-35133 Romagne (FR); VOLLE, Isabelle, F-69007 Lyon (FR)
(74) Mandataire: L'Helgoualch, Jean
(86) Numéro de dépôt international: PCT/FR2010/052165
(87) Numéro de publication internationale: WO 2011/051591

(56) Documents cités:
- DE-A1-102008 012 457
- Ephyla: "Revertime - Anti rides, Anti âge", , 3 avril 2008 (2008-04-03), XP002619147, Extrait de l'Internet: URL:http://www.cosmetane.com/media/pdf/Rev ertime_2008_04_03.pdf [extrait le 2011-01-28]

## Description

La présente invention se rapporte à une composition dermatologique et/ou cosmétique destinée à lutter efficacement contre le vieillissement de la peau, et plus particulièrement à régénérer le derme et l'épiderme de la peau.

La peau constitue tout à la fois une barrière anatomique vivante et une zone d'échange entre le corps et son environnement, et l'efficacité de cette barrière conditionne le maintien d'un bon équilibre homéostasique.

Elle comprend plusieurs couches allant de la couche superficielle, l'épiderme, aux couches plus profondes, le derme et l'hypoderme. Chacune de ces couches possède des propriétés spécifiques permettant à l'ensemble de réagir et de s'adapter aux conditions de son environnement.

L'épiderme, qui est composé de trois types de cellules, à savoir des kératinocytes (90% des cellules épidermiques), des mélanocytes (2 à 3% des cellules épidermiques) et des cellules de Langerhans, constitue la couche externe et joue un rôle fondamental pour assurer la protection et le maintien d'une bonne trophicité. Le derme est 10 à 30 fois plus épais que l'épiderme, auquel il sert de support, et il est principalement constitué de fibroblastes et d'une matrice extracellulaire essentiellement à base de collagène et d'élastine. Les fibres de collagène, qui représentent environ 70% des protéines du derme, contribuent à la texture et à la tonicité de la peau, tandis que l'élastine est responsable de son élasticité. D'autres cellules, comme les macrophages et les leucocytes, sont également présentes dans la couche du derme. L'hypoderme, qui est la couche la plus profonde de la peau, contient les adipocytes qui produisent des lipides pour que le tissu sous-cutané fabrique une couche grasse protégeant les muscles, les os, et les organes internes contre les chocs.

La jonction dermo-épidermique est la zone de rattachement de l'épiderme au derme, en particulier par le réseau de fibres de collagène, qui contrôle les échanges entre ces deux couches. Elle constitue le support des kératinocytes lors du processus de cicatrisation.

Les premiers signes du vieillissement de la peau, tels que les rides et ridules, sont généralement provoqués par le stress et les changements biologiques et physiologiques, accélérés par les modes de vie ou par l'environnement, en particulier la pollution, l'exposition au soleil, aux rayons ultraviolets, les variations de températures et les radicaux libres, qui peuvent contribuer à accélérer la dégradation des constituants principaux de la matrice extracellulaire du derme tels que le collagène ou l'acide hyaluronique. L'apparition de marques pigmentaires, la diminution de l'épaisseur de la peau, sa perte d'élasticité et son affaissement sont également des changements observés au cours du vieillissement.

On sait que la capacité de la peau à remplacer le collagène endommagé diminue avec le temps, et en conséquence des espaces et des irrégularités apparaissent dans le réseau du collagène.

L'acide hyaluronique, quant à lui, joue un rôle important dans la croissance cellulaire, la fonction des récepteurs membranaires et l'adhésion des cellules. A partir d'un certain âge, on note une diminution physiologique de la concentration d'acide hyaluronique dans la peau.

De nombreuses études ont été consacrées au traitement et à la prévention des signes du vieillissement cutané, notamment pour mettre au point des compositions dermatologiques et/ou cosmétiques susceptibles de favoriser la restructuration tissulaire, et en particulier la néosynthèse d'éléments constitutifs de la peau comme le collagène et l'acide hyaluronique de la matrice extracellulaire. Par "néosynthèse", on entend la synthèse du collagène ou de l'acide hyaluronique qui se forme uniquement en cas de besoin, par exemple pour le comblement des rides.

Par ailleurs, on sait que, pour être acceptées par les utilisateurs, les compositions topiques utilisables en dermatologie et/ou cosmétique doivent être agréables à utiliser et doivent présenter de bonnes propriétés physiques, notamment de consistance et d'onctuosité, tout en garantissant une efficacité satisfaisante et en évitant les inconvénients tels que tiraillements, irritations ou démangeaisons, que peuvent occasionner certains principes actifs.

Dans l'état de la technique, le brevet FR-A-2.783.169 décrit l'utilisation de pentapeptides du type Lys-Thr-Thr-Lys-Ser dans des compositions topiques pour favoriser la synthèse du collagène et des glycosaminoglycannes, et par conséquent la régénération cutanée. On connaît également, du brevet FR-A-2.848.116, une composition cosmétique et/ou dermatologique à base d'inhibiteurs de métallo-protéinases matricielles, et de lipopeptides permettant le traitement et la prévention des signes du vieillissement cutané tels que l'apparition de rides et la perte d'élasticité de la peau.

On peut également citer les brevets suivantes :
- le brevet DE 10 2008 012457, qui décrit une formulation de type crème associant un tripeptide avec du disteardimonium hectorite ;
- le brevet US 2006/104931, qui décrit une composition cosmétique comprenant, entre autres, de la laponite, un lipopeptide et du thé vert ; et
- le brevet US 2009/117061, qui décrit une composition pour la peau à base d'hectorite et d'un tétrapeptide.

Cependant, malgré les diverses compositions dermatologiques et/ou cosmétiques disponibles, il existe toujours un besoin de pouvoir disposer de nouvelles compositions topiques alternatives permettant de lutter efficacement contre les effets du vieillissement cutané, et notamment des compositions à base de substances d'origine naturelle adaptées au traitement et aux soins de la peau.

La présente invention vise à fournir notamment une composition dermatologique et/ou cosmétique permettant de lutter efficacement contre le vieillissement de la peau, et plus particulièrement permettant de régénérer la peau tel que le derme et l'épiderme.

La présente invention a pour objet une nouvelle composition topique utilisable en dermatologie et/ou en cosmétique comprenant l'association d'au moins un lipopeptide et d'au moins une argile fonctionnalisée.

L'invention a aussi pour objet l'utilisation cosmétique d'une composition associant au moins un lipopeptide et au moins une argile fonctionnalisée pour régénérer la peau.

Un autre objet de l'invention concerne l'utilisation d'une composition associant au moins un lipopeptide et au moins une argile fonctionnalisée pour la fabrication d'un produit dermatologique, cette utilisation étant préférentiellement destinée à la régénération de la peau.

L'association de l'invention présente l'avantage de vectoriser, ou d'intercaler, le ou les lipopeptides au sein de l'argile fonctionnalisée. On peut ainsi parler d'une composition topique utilisable en dermatologie et/ou en cosmétique comprenant au moins un lipopeptide intercalé dans une argile fonctionnalisée.

L'argile fonctionnalisée utilisée dans l'invention est obtenue à partir d'une argile et d'un composé fonctionnalisant (ladite argile).

L'argile en tant que telle utilisée pour obtenir l'argile fonctionnalisée est de préférence une argile comportant une structure feuilletée ou en feuillets. Mais ce peut être également un autre type d'argile comme par exemple une argile comportant une structure fibreuse.

Plus particulièrement, le ou les lipopeptides peuvent s'intercaler entre les feuillets de l'argile, typiquement appelé « espace inter-feuillets ». En d'autres termes, le ou les lipopeptide(s) intercalé(s) se trouvent à l'intérieur des feuillets, ce qui les protègent des agressions du milieu extérieur.

A titre d'exemple, une argile comportant une structure en feuillets peut appartenir à la famille des phyllosilicates. Ainsi, le ou les lipopeptides peuvent s'intercaler entre les feuillets de phyllosilicate, et se lier audits feuillets par liaisons de Van der Waals d'une part, et par substitution cationique d'autre part.

Les phyllosilicates peuvent être choisis parmi les montmorillonites, la beidellite, la saponite, les illites, la glauconite, les chlorites, la vermiculite, et les argiles fibreuses, ou un de leurs mélanges. Parmi les phyllosilicates cités, on préférera utiliser les montmorillonites.

Le composé fonctionnalisant peut être un composé glycoprotéique comme par exemple un extrait d'algue, et de préférence un extrait d'algue verte Ulva lactuca. Ainsi, le ou les lipopeptides peuvent se lier notamment par liaisons hydrogènes audit composé glycoprotéique, et conférer une très bonne stabilité à la structure composé du ou des lipopeptide(s) intercalé(s) dans l'argile fonctionnalisée.

Une argile fonctionnalisée utilisée dans la composition de l'invention peut être le produit référencé Revertime^{®}, commercialisé par la société Ephyla³. Ce produit est un composé naturel actif hybride, sous forme de poudre, issue d'une argile Montmorillonite et d'une algue verte.

Divers lipopeptides peuvent être utilisés dans le cadre de la présente invention.

Suivant une forme de réalisation de l'invention, le lipopeptide peut être choisi parmi un lipotripeptide, un lipotétrapeptide, un lipopentapeptide, et un lipohexapeptide.

A titre d'exemple, on peut citer :
- le N-palmitoyl-Gly-His-Lys comme tripeptide,
- le N-palmitoyl-Gly-Glu-Pro-Arg comme tétrapeptide,
- le N-palmitoyl-Lys-Thr-Thr-Lys-Ser comme pentapeptide, qui est commercialisé par la société Sederma sous la référence Matrixyl^{®}, ou
- le N-palmitoyl-Val-Gly-Val-Ala-Pro-Gly comme lipohexapeptide, qui est commercialisé par la société Sederma sous la référence Dermaxyl^{®}.

Ces peptides peuvent être combinés entre eux, et par exemple, dans la composition selon l'invention, on peut utiliser l'association d'un lipotripeptide tel que le N-palmitoyl-Gly-His-Lys, et d'un lipotetrapeptide tel que le N-palmitoyl-Gly-Glu-Pro-Arg. On préférera une composition selon l'invention comprenant au moins deux lipopeptides.

L'association du N-palmitoyl-Gly-His-Lys et du N-palmitoyl-Gly-Glu-Pro-Arg est commercialisée par la société Sederma sous la référence Matrixyl^{®} 3000.

Selon un mode de réalisation particulier de l'invention, la composition peut comprendre au plus 0,1% en poids de lipopeptide(s), et de préférence au plus 0,08% en poids de lipopeptide(s), et encore plus préférentiellement au plus 0,075% en poids de lipopeptide(s). La quantité particulièrement préférée dans la composition de l'invention est de 0,045% en poids de lipopeptide(s).

En outre, il est préférable que la composition comprenne au moins 0,015% en poids de lipopeptide(s).

Dans un autre mode de réalisation particulier, la composition peut comprendre au plus 1 % en poids d'argile(s) fonctionnalisée(s), de préférence au plus 0,1% en poids d'argile(s) fonctionnalisé(s). La quantité particulièrement préférée dans la composition de l'invention est de 0,01% en poids d'argile(s) fonctionnalisé(s).

La limite supérieure de 1% en poids présente l'avantage de permettre de solubiliser facilement l'argile fonctionnalisée dans la composition conforme à l'invention. En outre, cette limite permet d'obtenir de bonnes propriétés organoleptiques, notamment en termes de couleur et d'odeur.

En outre, il est préférable que la composition comprenne au moins 0,001% en poids d'argile(s) fonctionnalisée(s).

Les essais effectués avec la composition de l'invention ont montré que la combinaison d'au moins un lipopeptide et d'au moins une argile fonctionnalisée procurait d'intéressantes propriétés, et en particulier une action significative sur la régénération du derme et de l'épiderme, comme indiqué plus en détails ci-après.

Les compositions conformes à la présente invention peuvent être présentées sous les formes classiquement utilisées pour une application topique, c'est-à-dire sous forme de gel, lotion, émulsion (en particulier crème ou lait), sérum (ou essence), masque ou pommade, contenant des excipients et supports usuels compatibles et pharmaceutiquement acceptables. Elles peuvent aussi se présenter sous forme de lingettes imbibées d'une solution contenant les extraits suivant l'invention.

Ces formes d'administration par voie topique sont préparées par les techniques connues, et par exemple, dans le cas d'une crème, par dispersion d'une phase grasse dans une phase aqueuse pour obtenir une émulsion huile dans eau, ou inversement pour préparer une émulsion eau dans huile.

Les compositions topiques selon l'invention peuvent comprendre divers excipients usuels adaptés à une administration topique externe, en particulier des excipients acceptables sur le plan dermatologique et cosmétologique. Ces excipients appropriés pour la formulation sont bien connus de l'homme du métier et peuvent être choisis par exemple parmi un ou plusieurs des composés suivantes :
- des émulsionnants et co-émulsionnants tels que arachidyl glucoside, cétéaryl glucoside, behenyl alcohol, cétéaryl alcohol, cétostéarylique alcohol, cétéaryl alcohol, steareth-2, steareth-21, isostearyl isostearate, ou myristate d'isopropyl ;
- des émollients tels que octyl dodecanol, isononyl isononanoate, capric/caprylic triglycérides ;
- des silicones telles que le diméthicone ;
- des humectants tels que la glycérine, le butylène glycol ;
- des anti-oxydants tels que l'acétate de tocophérol ;
- des conservateurs tels que le phénoxyéthanol, l'acide déhydroacétique, l'acide para-hydroxybenzoïque et ses sels, l'acide benzoïque, le sorbate de potassium, le benzoate de sodium ;
- des poudres exfoliantes, des poudres de fruits ou des poudres matifiantes telles que le copolymère de méhacrylate de méthyle;
- des agents gélifiants ;
- des agents neutralisants ;
- des solubilisants ; et/ou
- des colorants.

Un autre objet de l'invention concerne un procédé de préparation d'une association telle que définie dans la composition de l'invention, caractérisé en ce qu'il comprend les étapes consistant à :
i. hydrater l'argile fonctionnalisée dans un milieu aqueux ou alcoolique pour obtenir une argile fonctionnalisée hydratée, et
ii. mélanger le ou les lipopeptide(s) et l'argile fonctionnalisée hydratée, pour obtenir un ou des lipopeptide(s) intercalé(s) dans l'argile fonctionnalisée.

L'étape i et l'étape ii peuvent être réalisées de façon concomitante lorsque ledit milieu comprend le ou les lipopeptide (s).

Grâce à l'étape i d'hydratation permettant de faire croître l'espace inter-feuillets (i.e. gonflement des feuillets) dans l'argile fonctionnalisée, l'intercalation du ou des lipopeptide(s) au sein de ladite argile fonctionnalisée peut être améliorée.

Ledit procédé peut comprendre en outre l'étape consistant à :
iii. sécher le ou les lipopeptide(s) intercalé(s) dans l'argile fonctionnalisée obtenu(s) à l'étape ii.

L'étape iii permet de fixer le ou les lipopeptide(s) à ladite argile fonctionnalisée.

L'association obtenue à partir dudit procédé de préparation permet ainsi d'obtenir de façon optimale des lipopeptides intercalés dans l'argile fonctionnalisée.

La composition de l'invention peut bien entendu comprendre ladite association obtenue à partir dudit procédé de préparation, et plus généralement comprendre au moins un lipopeptide et au moins une argile fonctionnalisée hydratée.

D'autres caractéristiques et avantages de la présente invention apparaîtront à la lumière des exemples qui vont suivre, lesdits exemples étant donnés à titre illustratif et nullement limitatif.

Des exemples de formulations sont rapportés aux exemples 1 et 2. Ces formulations sont à base du produit « Revertime » et du produit « Matrixyl^{®} 3000 ». Les quantités des différents constituants des formulations de ces deux exemples sont exprimées en % en poids. L'eau déminéralisée est présente dans la formulation finale en quantité suffisante pour (abrégé par le sigle « q.s.p. ») que le poids final de ladite formulation soit atteint, sans le dépasser (i.e. 100% en poids).

Le produit Revertime^{®} est uniquement constitué de ladite argile fonctionnalisée, et comprend environ 20% de matière organique (i.e. algue verte) pour 80% de matière inorganique (i.e. argile Montmorillonite).

Le produit Matrixyl^{®} 3000 comprend 50 ppm de lipotétrapeptide (i.e. N-palmitoyl-Gly-Glu-Pro-Arg) et 100 ppm de lipotripeptide (i.e. N-palmitoyl-Gly-His-Lys).

Dans la présente description, l'abréviation « ppm » signifie « parties par million massiques». En d'autres termes, la quantité (ou la teneur) x en ppm d'un composé z est exprimée par rapport au poids total de la formulation ou du produit considéré.

Les exemples 3 et 4 sont relatifs à des formulations alternatives mises en oeuvre sur la base des formulations des exemples 1 et 2.

L'exemple 5 est relatif à l'évaluation de la cytotoxicité du produit Revertime^{®} vis-à-vis de fibroblastes dermiques humains normaux cultivés en monocouches.

L'exemple 6 est relatif à l'évaluation de la cytotoxicité des deux lipopeptides du produit Matrixyl^{®} 3000 vis-à-vis de fibroblastes dermiques humains normaux cultivés en monocouches.

L'exemple 7 est relatif à l'étude de l'effet de l'association entre le produit Revertime^{®}, et les deux lipopeptides du produit Matrixyl^{®} 3000, sur la néosynthèse de procollagène de type I et d'acide hyaluronique dans un modèle de fibroblastes dermiques humains normaux adultes cultivés en monocouches.

### Exemple 1

Suivant les techniques classiques, on prépare un gel à base d'une composition selon l'invention pour régénérer la peau ayant la composition pondérale suivante :

| | |
|---|---|
| Eau déminéralisée, q.s.p. | 100 |
| Agent gélifiant | 3 |
| Agent neutralisant | 3 |
| Glycols | 5 |
| Glycérine | 5 |
| Conservateurs | 3 |
| Colorants | 0,1 |
| Solubilisant | 3 |
| Parfum | 0,5 |
| Actifs divers (hydratant et/ou lissant) | 20 |
| Revertime^{®} | 0,1 |
| Matrixyl^{®} 3000 | 3 |

### Exemple 2

Suivant les techniques classiques, on prépare une émulsion à base d'une composition selon l'invention pour régénérer la peau ayant la composition pondérale suivante :

| | |
|---|---|
| Eau déminéralisée, q.s.p. | 100 |
| Agent gélifiant | 1 |
| Glycérine | 5 |
| Conservateurs | 3 |
| Emulsionnant | 5 |
| Co-Emulsionnant | 5 |
| Esters, huiles | 20 |
| Silicone | 3 |
| Actifs divers (hydratant et/ou lissant) | 20 |
| Colorants | 0,1 |
| Parfum | 0,5 |
| Revertime^{®} | 0,1 |
| Matrixyl^{®} 3000 | 3 |

Ces formulations (gel de l'exemple 1 et émulsion de l'exemple 2) permettent de lutter efficacement contre le vieillissement de la peau.

### Exemple 3

L'efficacité des formulations des exemples 1 et 2 peut être optimisée en purifiant le produit Matrixyl^{®} 3000, afin d'extraire les deux lipopetides qui le composent.

En effet, le produit Matrixyl^{®} 3000 est constitué des deux lipopeptides mélangés à un milieu glycériné gélifié avec un carbomère.

Pour se faire, on peut procéder à l'extraction des deux lipopeptides qui composent le produit Matrixyl^{®} 3000 selon les étapes suivantes :
- précipiter le carbomère, par exemple en mélangeant sous agitation le Matrixyl^{®} 3000 avec une solution éthanolique, et
- séparer le carbomère précipité à l'étape précédente pour obtenir les deux lipopeptides sous forme de filtrat (Filtrat A).

Le Filtrat A peut alors être directement utilisé dans les formulations des exemples 1 et 2 à la place du produit Matrixyl^{®} 3000, dans des concentrations de lipopeptides telles que mentionnées dans la présente description.

### Exemple 4

Avant l'incorporation du Filtrat A dans les formulations des exemples 1 et 2, on peut également améliorer l'intercalation des deux lipopeptides au sein de l'argile fonctionnalisée (i.e. Revertime^{®}) en mélangeant, par exemple pendant 2h, de préférence à l'obscurité, le Filtrat A et l'argile fonctionnalisée, permettant ainsi de bien hydrater l'argile fonctionnalisée.

Après filtration du mélange constitué du Filtrat A et de l'argile fonctionnalisée, on procède au séchage du résidu solide.

Le résidu solide séché peut alors être directement utilisé dans les formulations des exemples 1 et 2 à la place du produit Matrixyl^{®} 3000 et du produit Revertime^{®}, dans des concentrations de lipopeptides telles que mentionnées dans la présente description.

### Exemple 5

### Etude de la cytotoxicité du produit Revertime^{®} vis-à-vis de fibroblastes dermiques humains normaux cultivés en monocouches

### 5.1 Matériels et méthodes

### 5.1.1. Système d'essai

Des fibroblastes dermiques humains normaux ont été obtenus à partir d'une plastie abdominale réalisée chez une femme de 35 ans. Pour la réalisation des essais, ces cellules ont été cultivées jusqu'à l'obtention de monocouches confluentes.

### 5.1.2. Incubation des cellules avec les produits à l'essai

Les cellules ont été incubées en absence (contrôle) ou en présence des concentrations croissantes du produit Revertime^{®}.

Le produit Revertime^{®} a été préparé selon le protocole suivant.

Le produit Revertime^{®} a été solubilisé dans du DMSO 100% à une concentration de 1% en poids (concentration finale après solubilisation dans le DMSO), le surnageant obtenu après une heure dans un bain-marie à 37°C et après ultrasons a été dilué dans le milieu d'incubation (concentration finale maximum en DMSO dans le milieu d'incubation = 1% en volume).

Les concentrations finales en poids de Revertime^{®} dans le milieu d'incubation, utilisées pour l'incubation des cellules, sont les suivantes : 0,000001 ; 0,00001 ; 0,00005 ; 0,0001 ; 0,0005 ; 0,001 ; 0,005 et 0,01 %.

Les cellules ont ensuite été incubées pendant 48 heures en absence (contrôle) ou en présence des concentrations croissantes, telles que mentionnées ci-avant, du produit Revertime^{®} « dilué » dans le milieu d'incubation.

### 5.1.3. Evaluation des effets

A la fin de la période d'incubation, la viabilité des cellules a été évaluée par une méthode spectrophotométrique de dosage de l'activité des phosphatases intracellulaires. (transformation du p-nitrophényl phosphate en p-nitrophénol par les phosphatases intracellulaires des cellules viables ; absorbance du p-nitrophénol à 405 nm directement proportionnelle au nombre des cellules viables présentes dans les puits de culture).

### 5.2. Résultats

Les résultats obtenus montrent que dans les conditions expérimentales retenues, après 48 heures d'incubation, le produit Revertime^{®} ne diminue pas significativement (>20%) le nombre de fibroblastes dermiques viables présents dans les puits de culture.

Par conséquent, le produit Revertime^{®} ne présente pas un effet cytotoxique vis-à-vis de fibroblastes dermiques normaux cultivés en monocouches.

### Exemple 6

### Etude de la cytotoxicité des deux lipopeptides du produit Matrixyl^{®} 3000 vis-à-vis de fibroblastes dermiques humains normaux cultivés en monocouches

### 6.1. Matériels et méthodes

### 6.1.1. Système d'essai

Des fibroblastes dermiques humains normaux ont été obtenus à partir d'une plastie abdominale réalisée chez une femme de 35 ans. Pour la réalisation des essais, ces cellules ont été cultivées jusqu'à l'obtention de monocouches confluentes.

### 6.1.2. Incubation des cellules avec les produits à l'essai

Les cellules ont été incubées en absence (contrôle) ou en présence de concentrations croissantes des deux lipopeptides du produit Matrixyl^{®} 3000.

Les deux lipopeptides (i.e. le N-palmitoyl-Gly-Glu-Pro-Arg et le N-palmitoyl-Gly-His-Lys), ou actifs, ont été préparés selon le protocole suivant.

L'actif « N-palmitoyl-Gly-Glu-Pro-Arg » a été solubilisé à 3,22 mg/ml dans une solution de DMSO / HCl à 37% (98,5:1,5), et l'actif « N-palmitoyl-Gly-His-Lys » a été solubilisé à 6,90 mg/ml en DMSO / HCl 37% (98,5:1,5) pour former respectivement deux solutions mères.

Ces deux solutions mères ont été vortexées, solubilisées à l'aide d'ultrasons et incubées une heure dans un bain-marie à 50°C. Elles ont ensuite été mélangées entre elles, à volumes équivalents, pour obtenir un « MIX ».

Les solutions d'essai ont ensuite été diluées dans le milieu d'incubation (concentration finale minimum de DMSO dans le milieu d'incubation = 0,1% en volume).

Les concentrations finales en poids de MIX dans le milieu d'incubation, utilisées pour l'incubation des cellules, sont les suivantes : 0,00045 ; 0,00075 ; 0,001125 ; 0,0015 ; 0,0045 ; 0,0075 ; 0,01125 ; 0,015 ; 0,0225 ; 0,045 ; 0,075 ; 0,1125 et 0,15 %.

Les cellules ont ensuite été incubées pendant 24 heures en absence (contrôle) ou en présence de concentrations croissantes, telles que mentionnées ci-avant, du MIX « dilué » dans le milieu d'incubation.

### 6.1.3. Evaluation des effets

La méthode utilisée est identique à celle décrite dans l'exemple 5 au paragraphe 5.1.3.

### 6.2. Résultats

Les résultats obtenus montrent que dans les conditions expérimentales retenues, après 24 heures d'incubation, l'association des deux lipopeptides du produit Matrixyl^{®} 3000 ne diminue pas significativement (>20%) le nombre de fibroblastes dermiques viables présents dans les puits de culture.

Par conséquent, l'association des deux lipopeptides du produit Matrixyl^{®} 3000 ne présentait pas d'effet cytotoxique vis-à-vis de fibroblastes dermiques normaux cultivés en monocouches aux concentrations testées (0,00045 à 0,15%).

### Exemple 7

### Etude de l'effet du produit Revertime^{®} et des deux lipopeptides du produit Matrixyl^{®} 3000 sur la néosynthèse de procollagène de type I et d'acide hyaluronique dans un modèle de fibroblastes dermiques humains normaux adultes cultivés en monocouches

### 7.1. Matériels et méthodes

### 7.1.1. Système d'essai

Des monocouches confluentes de fibroblastes dermiques humains normaux ont été obtenues en cultivant des cellules issues d'une plastie abdominale réalisée chez une femme de 33 ans.

L'activateur de référence utilisé a été le « *Transforming Growth Factor β* » (TGF-β) à 10 ng/ml.

### 7.1.2. Incubation des produits

Les fibroblastes ont été incubés 24 heures (pour l'étude de l'effet des produits à l'essai sur la production d'acide hyaluronique) et 48 heures (pour l'étude de l'effet des produits à l'essai sur la production de procollagène de type I), à 37°C, sous atmosphère humide et 5% de CO₂, en absence (milieu de culture seul) ou en présence du produit de référence, ou de concentrations croissantes des produits a l'essai.

L'incubation a été réalisée sur les produits suivants :
- le produit Revertime^{®} à 0,001 ; 0,003 et 0,01 % (p/v) ;
- les deux lipopeptides du produit Matrixyl^{®} 3000 (« MIX ») à 0,015 ; 0,045 et 0,075 % ;
- Association 1 : Revertime^{®} à 0,003% + MIX à 0,015% ;
- Association 2 : Revertime^{®} à 0,01% + MIX à 0,045% ; et
- Association 3 : Revertime^{®} à 0,01% + MIX à 0,075%.

Le produit Revertime^{®} a été préparé selon un protocole identique à celui décrit dans l'exemple 5 au paragraphe 5.1.2.

Les deux lipopeptides du produit Matrixyl^{®} 3000 pour former le « MIX » ont été préparés selon un protocole identique à celui décrit dans l'exemple 6 au paragraphe 6.1.2.

### 7.1.3. Evaluation des effets

### - Dosage du procollagène de type I et de l'acide hyaluronique

A la fin de la période d'incubation, le procollagène de type I et l'acide hyaluronique contenus dans les milieux de culture ont été quantifiés a l'aide de kits E.L.I.S.A. sensibles et spécifiques.

### - Dosage des protéines

A la fin de la période d'incubation, les protéines contenues dans les lysats cellulaires ont été quantifiées par une méthode spectrocolorimétrique (méthode de Bradford).

### 7.2. Résultats

### 7.2.1. Produit Revertime^{®}

Les résultats sont rassemblés dans le tableau 1 ci-après.

**Tableau 1**

| | | Néosynthèse de procollagène de type I | Néosynthèse d'acide hyaluronique |
|---|---|---|---|
| Revertime^{®} | 0,001% | + 21,8% | - 14,4% |
| | 0,003% | + 40,9% | - 24,9% |
| | 0,01% | - 2,2% | - 8,6% |

Dans les conditions expérimentales retenues, le produit Revertime^{®} induit une augmentation significative de la néosynthèse de procollagène de type I, notamment pour le produit Revertime^{®} à 0,001 % et à 0,003 %. Toutefois, il n'induit aucune augmentation significative de la néosynthèse d'acide hyaluronique.

### 7.2.2. Les deux lipopeptides du produit Matrixyl^{®} 3000

Les résultats sont rassemblés dans le tableau 2 ci-après.

**Tableau 2**

| | | Néosynthèse de procollagène de type I | Néosynthèse d'acide hyaluronique |
|---|---|---|---|
| MIX | 0,015% | - 15,1% | + 16,0% |
| | 0,045% | - 15,9% | + 43,8% |
| | 0,075% | + 17,0% | + 33,8% |

Dans les conditions expérimentales retenues, l'association des deux lipopeptides du produit Matrixyl^{®} 3000 n'induit pas d'augmentation significative de la néosynthèse de procollagène de type I avec une production de + 17,0 % pour ladite association à 0,075%. Toutefois, il induit une augmentation significative de la néosynthèse d'acide hyaluronique.

### 7.2.3. Association du produit Revertime^{®} et des deux lipopeptides du produit Matrixyl^{®} 3000

Les résultats sont rassemblés dans le tableau 3 ci-après.

**Tableau 3**

| | | Néosynthèse de procollagène de type I | Néosynthèse d'acide hyaluronique |
|---|---|---|---|
| Association | 1 | + 75,2% | - 10,0% |
| | 2 | + 49,6% | + 78,7% |
| | 3 | + 48,9% | + 32,3% |

Dans les conditions expérimentales retenues, l'association du produit Revertime^{®} et des deux lipopeptides du produit Matrixyl^{®} 3000 induit d'une part, une augmentation significative de la néosynthèse de procollagene de type I (Associations 1, 2 et 3), et d'autre part, une augmentation significative de la néosynthèse d'acide hyaluronique (Associations 2 et 3).

### 7.3. Conclusion

Les effets de l'association du produit Revertime (argile fonctionnalisée) et des deux lipopeptides du produit Matrixyl^{®} 3000 sont ainsi significativement supérieurs à ceux obtenus en présence de l'utilisation seule du produit Revertime^{®} ou de l'association des deux lipopeptides du produit Matrixyl^{®} 3000, que ce soit sur la néosynthèse de procollagène de type I ou sur la néosynthèse d'acide hyaluronique. Ces effets sont également supérieurs à la somme des effets obtenus en présence de chacun des produits à l'essai (produit Revertime^{®} d'une part, et association des deux lipopeptides du produit Matrixyl^{®} 3000 d'autre part) utilisés séparément.

Ces résultats permettant avantageusement de montrer l'existence d'une réelle synergie d'action de l'association d'au moins une argile fonctionnalisée et d'au moins un lipopeptide sur les paramètres mesurés (production de procollagène de type I et production d'acide hyaluronique).

## Revendications

1. Composition topique utilisable en dermatologie et/ou en cosmétique, **caractérisée en ce qu'**elle comprend l'association d'au moins un lipopeptide et d'au moins une argile fonctionnalisée, l'argile fonctionnalisée étant obtenue à partir d'une argile et d'un composé fonctionnalisant du type composé glycoprotéique.

2. Composition selon la revendication 1, **caractérisée en ce que** l'argile comporte une structure en feuillets.

3. Composition selon la revendication 1 ou 2, **caractérisée en ce que** l'argile appartient à la famille des phyllosilicates.

4. Composition selon la revendication 3, **caractérisée en ce que** les phyllosilicates sont choisis parmi les montmorillonites, la beidellite, la saponite, les illites, la glauconite, les chlorites, la vermiculite, et les argiles fibreuses, ou un de leurs mélanges.

5. Composition selon l'une quelconques des revendications précédentes, **caractérisée en ce que** le composé glycoprotéique est un extrait d'algue.

6. Composition selon la revendication 5, **caractérisée en ce que** l'extrait d'algue est un extrait d'algue verte Ulva lactuca.

7. Composition selon l'une quelconque des revendications précédentes, **caractérisée en ce que** le lipopeptide est choisi parmi un lipotripeptide, un lipotétrapeptide, un lipopentapeptide et un lipohexapeptide.

8. Composition selon l'une quelconque des revendications précédentes, **caractérisée en ce que** le lipopeptide est choisi parmi le N-palmitoyl-Gly-His-Lys, le N-palmitoyl-Gly-Glu-Pro-Arg, le N-palmitoyl-Lys-Thr-Thr-Lys-Ser, et le N-palmitoyl-Val-Gly-Val-Ala-Pro-Gly.

9. Composition selon l'une quelconque des revendications précédentes, **caractérisée en ce que** la composition comprend deux lipopeptides.

10. Composition selon l'une quelconque des revendications précédentes, **caractérisée en ce que** la composition comprend un lipotripeptide et un lipotétrapeptide.

11. Composition selon la revendication 10, **caractérisée en ce que** la composition comprend le N-palmitoyl-Gly-His-Lys et le N-palmitoyl-Gly-Glu-Pro-Arg.

12. Composition selon l'une quelconque des revendications précédentes, **caractérisée en ce qu'**elle comprend au plus 0,1% en poids de lipopeptide(s).

13. Composition selon l'une quelconque des revendications précédentes, **caractérisée en ce qu'**elle comprend au plus 1 % en poids d'argile(s) fonctionnalisée(s).

14. Procédé de préparation d'une association telle que définie dans la composition selon les revendications 1 à 13, **caractérisé en ce qu'**il comprend les étapes consistant à :
i. hydrater l'argile fonctionnalisée dans un milieu aqueux ou alcoolique pour obtenir une argile fonctionnalisée hydratée, et
ii. mélanger le ou les lipopeptide(s) et l'argile fonctionnalisée hydratée, pour obtenir un ou des lipopeptide(s) intercalé(s) dans l'argile fonctionnalisée.

15. Procédé selon la revendication 14, **caractérisée en ce qu'**il comprend en outre l'étape consistant à :
iii. sécher le ou les lipopeptide(s) intercalé(s) dans l'argile fonctionnalisée obtenu(s) à l'étape ii.

16. Utilisation cosmétique de la composition selon l'une quelconque des revendications 1 à 13, pour régénérer la peau.

17. Utilisation de la composition selon l'une quelconque des revendications 1 à 13, pour la fabrication d'un produit dermatologique.

## Patentansprüche

1. Topische Zusammensetzung, die in der Dermatologie und/ oder in der Kosmetik verwendbar ist, **dadurch gekennzeichnet, dass** sie die Kombination mindestens eines Lipopeptids und mindestens eines funktionalisierten Tons umfasst, wobei der funktionalisierte Ton aus einem Ton und einer funktionalisierenden Verbindung vom Typ Glykoproteinverbindung hergestellt ist.

2. Zusammensetzung nach Anspruch 1, **dadurch gekennzeichnet, dass** der Ton eine Blattstruktur aufweist.

3. Zusammensetzung nach Anspruch 1 oder 2, **dadurch gekennzeichnet, dass** der Ton zur Familie der Phyllosilikate gehört.

4. Zusammensetzung nach Anspruch 3, **dadurch gekennzeichnet, dass** die Phyllosilikate aus den Montmorilloniten, dem Beidellit, dem Saponit, den Illiten, dem Glaukonit, den Chloriten, dem Vermiculit und den faserigen Tonen oder ihren Gemischen ausgewählt sind.

5. Zusammensetzung nach einem der vorangehenden Ansprüche, **dadurch gekennzeichnet, dass** die Glykoproteinverbindung ein Algenextrakt ist.

6. Zusammensetzung nach Anspruch 5, **dadurch gekennzeichnet, dass** der Algenextrakt ein Extrakt der Grünalge Ulva lactuca ist.

7. Zusammensetzung nach einem der vorangehenden Ansprüche, **dadurch gekennzeichnet, dass** das Lipopeptid aus einem Lipotripeptid, einem Lipotetrapeptid, einem Lipopentapeptid und einem Lipohexapeptid ausgewählt ist.

8. Zusammensetzung nach einem der vorangehenden Ansprüche, **dadurch gekennzeichnet, dass** das Lipopeptid aus dem N-palmitoyl-Gly-His-Lys, dem N-palmitoyl-Gly-Glu-Pro-Arg, dem N-palmitoyl-Lys-Thr-Thr-Lys-Ser und dem N-palmitoyl-Val-Gly-Val-Ala-Pro-Gly ausgewählt ist.

9. Zusammensetzung nach einem der vorangehenden Ansprüche, **dadurch gekennzeichnet, dass** die Zusammensetzung zwei Lipopeptide umfasst.

10. Zusammensetzung nach einem der vorangehenden Ansprüche, **dadurch gekennzeichnet, dass** die Zusammensetzung ein Lipotripeptid und ein Lipotetrapeptid umfasst.

11. Zusammensetzung nach Anspruch 10, **dadurch gekennzeichnet, dass** die Zusammensetzung das N-palmitoyl-Gly-His-Lys und das N-palmitoyl-Gly-Glu-Pro-Arg umfasst.

12. Zusammensetzung nach einem der vorangehenden Ansprüche, **dadurch gekennzeichnet, dass** sie höchstens 0,1 Gew.-% Lipopeptid(e) umfasst.

13. Zusammensetzung nach einem der vorangehenden Ansprüche, **dadurch gekennzeichnet, dass** sie höchstens 1 Gew.-% funktionalisierte(n) Ton(e) umfasst.

14. Herstellungsverfahren einer wie in der Zusammensetzung gemäß den Ansprüchen 1 bis 13 definierten Kombination, **dadurch gekennzeichnet, dass** es die folgenden Schritte umfasst:
i. Hydratisieren des funktionalisierten Tons in einem wässrigen oder alkoholischen Milieu, um einen hydratisierten funktionalisierten Ton zu erhalten, und
ii. Mischen des oder der Lipopeptids(e) und des hydratisierten funktionalisierten Tons, um ein oder Lipopeptid(e), eingeschoben in den funktionalisierten Ton, zu erhalten.

15. Verfahren nach Anspruch 14, **dadurch gekennzeichnet, dass** es ferner einen folgenden Schritt umfasst:
iii. Trocknen des oder der Lipopeptids(e) aus Schritt ii, das oder die in den funktionalisierten Ton eingeschoben ist/sind.

16. Kosmetische Verwendung der Zusammensetzung nach einem der Ansprüche 1 bis 13 zur Regenerierung der Haut.

17. Verwendung der Zusammensetzung nach einem der Ansprüche 1 bis 13 zur Herstellung eines dermatologischen Produkts.

## Claims

1. A topical composition which may be used in dermatology and/or in cosmetics, **characterized in that** it comprises the combination of at least one lipopeptide and of at least one functionalized clay, the functionalized clay being obtained from clay and from a functionalizing compound of the glycoprotein compound type.

2. The composition according to claim 1, **characterized in that** the clay includes a sheet-like structure.

3. The composition according to claim 1 or 2, **characterized in that** the clay belongs to the family of phyllosilicates.

4. The composition according to claim 3, **characterized in that** the phyllosilicates are selected from montmorillonites, beidellite, saponite, illites, glauconite, chlorites, vermiculites, and fibrous clays, or one of their mixtures.

5. The composition according to any other preceding claims, **characterized in that** the glycoprotein compound is an algae extract.

6. The composition according to claim 5, **characterized in that** the algae extract is an extract of the green algae *Ulva lactuca.*

7. The composition according to any of the preceding claims, **characterized in that** the lipopeptide is selected from a lipotripeptide, a lipotetrapeptide, a lipopentapeptide and a lipohexapeptide.

8. The composition according to any of the preceding claims, **characterized in that** the lipopeptide is selected from N-palmitoyl-Gly-His-Lys, N-palmitoyl-Gly-Glu-Pro-Arg, N-palmitoyl-Lys-Thr-Thr-Lys-Ser, and N-palmitoyl-Val-Gly-Val-Ala-Pro-Gly.

9. The composition according to any of the preceding claims, **characterized in that** the composition comprises two lipopeptides.

10. The composition according to any of the preceding claims, **characterized in that** the composition comprises a lipotripeptide and a lipotetrapeptide.

11. The composition according to claim 10, **characterized in that** the composition comprises N-palmitoyl-Gly-His-Lys and N-palmitoyl-Gly-Glu-Pro-Arg.

12. The composition according to any of the preceding claims, **characterized in that** it comprises at most 0.1% by weight of lipopeptide(s).

13. The composition according to any of the preceding claims, **characterized in that** it comprises at most 1% by weight of functionalized clay(s).

14. A method for preparing a combination as defined in the composition according to claims 1 to 13, **characterized in that** it comprises the steps:
i. hydrating the functionalized clay in an aqueous or alcoholic medium in order to obtain a functionalized clay hydrate, and
ii. mixing the lipopeptide(s) and the functionalized clay hydrate, in order to obtain lipopeptide(s) inserted into the functionalized clay.

15. The method according to claim 14, **characterized in that** it further comprises the step:
iii. drying the lipopeptide(s) inserted into the functionalized clay obtained in step ii.

16. The cosmetic use of the composition according to any of claims 1 to 13, for regenerating skin.

17. The use of the composition according to any of claims 1 to 13, for manufacturing a dermatological product.
